# EUROPÄISCHE PATENTSCHRIFT

(11) **EP 0 457 155 B1**
(45) Veröffentlichungstag und Bekanntmachung des Hinweises auf die Patenterteilung: **15.11.1995**
(21) Anmeldenummer: 91107360.9
(22) Anmeldetag: 07.05.1991
(51) Int. Cl.: C07H 15/04, C07H 15/08, C11D 1/08

(54) **Alkylmono- und Alkylpolyglucosidether-carboxylate, Verfahren zu ihrer Herstellung und ihre Verwendung**
Carboxylate-ether derivatives of alkylmono- and alkylpolyglucosides, process for their preparation and their use
Ethers carboxylates d'alkylmono- et alkylpolyglucosides, procédé pour leur préparation et leur utilisation

(30) Priorität: 16.05.1990 DE 4015655
(43) Veröffentlichungstag der Anmeldung: 21.11.1991
(73) Patentinhaber: BASF Aktiengesellschaft, 67063 Ludwigshafen (DE)
(72) Erfinder: Oftring, Alfred, Dr., W-6702 Bad Duerkheim (DE); Kappes, Elisabeth, Dr., W-6700 Ludwigshafen (DE); Baur, Richard, Dr., W-6704 Mutterstadt (DE); Kud, Alexander, Dr., W-6509 Eppelsheim (DE)

(56) Entgegenhaltungen:
- EP-A- 0 258 814
- FR-A- 2 240 950
- FR-A- 2 240 950
- PHYTOCHEMISTRY Bd. 29, Nr. 2, OXFORD UK Seiten 513 - 515; Hermansson K; Kenne L; Rukunga G M; Samuelsson: 'Isolat on and characterization of 2-O-.beta.-D-glucopyranosyl-L-malic acid from Synade ium pereskiifolium'
- PATENT ABSTRACTS OF JAPAN vol. 12, no. 275 (C-516)(3122) 29. Juli 1988 & JP-A- 63 054 390 ( KAWAKEN FINE CHEM CO LTD ) 8. März 1988

## Beschreibung

Die Erfindung betrifft Alkylmono- und Alkylpolyglucosidethercarboxylate, ihre Herstellung und ihre Verwendung als Zusatz zu phosphatfreien und phosphatarmen Waschmitteln.

Aus der US-PS 4,524,009 und der US-PS 4,639,325 sind Glycerinethersuccinate bekannt, die durch Umsetzung von Glycerin mit Maleinsäure oder Maleinsäureanhydrid in Gegenwart von Erdalkalimetallhydroxyden bei Temperaturen oberhalb von 50°C und pH-Werten >10 hergestellt werden. An 1 Mol Glycerin können dabei 1 bis 3 Mol Maleinsäure unter Bildung der entsprechenden Glycerinethersuccinate angelagert werden. Die Glycerinethersuccinate werden als Substitutionsprodukte für Phosphor bzw. Stickstoff enthaltende Builder in Naschmitteln eingesetzt. Die Glycerinethercarboxylate sind biologisch abbaubar.

Aus der US-PS 4,663,071 sind Builder auf Basis von Ethercarboxylaten bekannt, die durch Anlagerung von 1 Mol bzw. 2 Mol Maleinsäure an 1 Mol Weinsäure in wäßriger Lösung in Gegenwart von Erdalkalimetall- und Alkalimetallionen erhältlich sind.

Aus der DE-OS 24 39 155 ist ein Verfahren zur Herstellung von Buildern bekannt, bei dem man beispielsweise an Monosaccharide oder Oligosaccharide α,β-ungesättigte Dicarboxylverbindungen, wie Maleinsäure, in Gegenwart von Erdalkalihydroxyden in wäßrigem Medium anlagert. Die so erhältlichen Builder sind biologisch abbaubar.

Hermansson et al. beschreiben in Phytochemistry, Band 29, 1990, Seiten 513-515, die Verbindung 2-O-β-D-Glucopyranosyl-L-Äpfelsäure. Hinweise auf mögliche Anwendungen im wasch- und Reinigungmittelsektor werden nicht gegeben.

Der vorliegenden Erfindung liegt die Aufgabe zugrunde, neue Stoffe zur Verfügung zu stellen.

Die Aufgabe wird erfindungsgemäß gelöst mit Alkylmono- und Alkylpolyglucosidethercarboxylaten der Formel 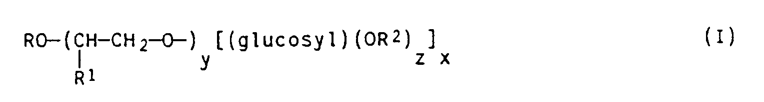
in der
- R =: C₁- bis C₂₄-Alkyl, C₁- bis C₂₄-Alkylphenyl, Hydroxy-C₂- bis C₆-alkyl, Hydroxy-C₁- bis C₂₄-alkylphenyl, 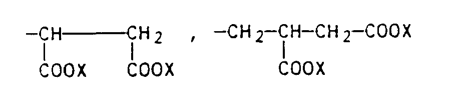 oder H,
- R¹=: H oder Methyl,
- R²=: 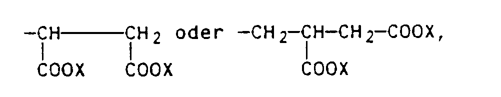
- X=: Wasserstoff-, Alkalimetall-, Ammonium- und/oder substituiertes Ammoniumäquivalent,
- x=: 1 bis 10,
- y=: 1 bis 10 und
- z=: 1 bis 4
bedeuten.

Die Alkylmono- und Alkylpolyglucosidethercarboxylate der Formel I sind dadurch erhältlich, daß man Alkylmono- oder Alkylpolyglucoside der Formel 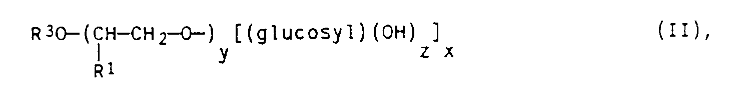
in der
- R³=: C₁- bis C₂₄-Alkyl, C₁- bis C₂₄-Alkylphenyl, Hydroxy-C₂- bis C₆-alkyl, Hydroxy-C₂- bis C₂₄-alkylphenyl oder Wasserstoff,
- R¹=: H oder Methyl,
- x=: 1 bis 10,
- y=: 1 bis 10 und
- z=: 1 bis 4
bedeuten, durch Reaktion mit Maleinsäure und/oder Itaconsäure in wäßrigem Medium in Gegenwart von mindestens 30 mol-% Erdalkalimetallionen, bezogen auf die genannten Dicarbonsäuren, bei pH-Werten von 9 bis 13 und Temperaturen von 50 bis 150°C zu Verbindungen der Formel I verethert.

Die Verbindungen der Formel II sind bekannte Stoffe. Alkylmonoglucoside der Formel II sind beispielsweise durch Umacetalisierung von Butylmonoglucosiden mit Ethylenglycol oder bis zu 10fach ethoxylierten C₁- bis C₂₄-Alkoholen der C₁- bis C₂₄-Alkylphenolen erhältlich. Alkylpolyglucoside, die beispielsweise 1,3 bis 10, vorzugsweise bis zu 3 Glucosideinheiten, enthalten, sind beispielsweise aus der EP-PS 0 075 995 bekannt. In Formel 11 bedeutet x vorzugsweise 1 bis 3, y vorzugsweise 1 bis 3 und z vorzugsweise eine Zahl von 3 bis 4. Als Verbindung der Formel II verwendet man vorzugsweise Hydroxyethylmonoglucosid, Hydroxyethylpolyglucosid mit 1,3 bis 3 Glucosideinheiten, Alkylmonoglucoside, die sich von C₁₀- bis C₁₂-Alkoholen oder deren Gemischen ableiten, Alkylmonoglucoside, die sich von 1 bis 10fach ethoxylierten oder propoxylierten C₁₀- bis C₁₂-Alkoholen ableiten sowie Alkylpolyglucoside von C₁₀- bis C₁₂-Alkoholen oder deren Gemischen mit 1,3 bis 3 Glucosideinheiten sowie die entsprechenden ethoxylierten und/oder propoxylierten Alkylpolyglucoside.

Die Veretherung der Verbindungen der Formel II erfolgt durch Umsetzung mit Maleinsäure, Itaconsäure oder Derivaten dieser Carbonsäuren, die unter den Reaktionsbedingungen Maleinsäure oder Itaconsäure bilden. Zu diesen Derivaten gehören Maleinsäureanhydrid, Itaconsäureanhydrid sowie die Halbester und Diester von Maleinsäure und Itaconsäure, z.B. Maleinsäuremonomethylester, Maleinsäuredimethylester, Maleinsäurediethylester, Itaconsäuremonomethylester und Itaconsäuredimethylester. Falls man bei der Veretherung die Ester der Dicarbonsäuren einsetzt, entstehen daraus im Reaktionsmedium die Dicarbonsäuren bzw. deren Salze sowie die den Estern zugrunde liegenden Alkohole. Aus den Säureanhydriden bilden sich in wäßrigem Medium die zugrunde liegenden Dicarbonsäuren bzw. die entsprechenden Salze der Dicarbonsäuren.

Die Umsetzung der Alkylmono- und Alkylpolyglucoside der Formel II mit Maleinsäure und/oder Itaconsäure erfolgt in wäßrigem Medium in Gegenwart von Erdalkalimetallionen, vorzugsweise in Gegenwart von Kalzium- und Bariumionen. Die Erdalkalimetallverbindungen werden vorzugsweise in Form der Oxide oder Hydroxide eingesetzt. Pro Mol Maleinsäure bzw. Itaconsäure, die umgesetzt werden sollen, verwendet man mindestens 30 bis 150 mol-% and Erdalkalimetallionen. Vorzugsweise beträgt die Menge an Erdalkalimetallionen 0,7 bis 1,3 mol, bezogen auf 1 mol der eingesetzten α,β-monoethylenisch ungesättigten Dicarboxylverbindungen. Der pH-Wert des Reaktionsmediums beträgt 9 bis 13, vorzugsweise 10 bis 11,5. Zur Herstellung der Verbindungen der Formel I, kann man beispielsweise so vorgehen, daß man eine wäßrige Lösung der Verbindungen der Formel II vorlegt, die notwendige Menge an Erdalkalimetallionen zufügt und die monoethylenisch ungesättigte Dicarboxylverbindung anschließend auf einmal, portionsweise oder kontinuierlich zugibt. Man kann jedoch auch die monoethylenisch ungesättigten Dicarboxylverbindungen zusammen mit der notwendigen Menge an Erdalkalimetallverbindung in wäßriger Lösung vorlegen und die Verbindungen der Formel II zugeben oder eine Mischung aus einer Verbindung der Formel II und Maleinsäure und/oder Itaconsäure herstellen und durch Zugabe von Erdalkalimetallverbindung und ggfs. einer anderen Base wie Natronlauge, Kalilauge oder tertiären Aminen die Reaktion starten. Während der Veretherung kann man zur Aufrechterhaltung des pH-Wertes in dem Bereich von 9 bis 13 die erforderliche Menge an Basen zufügen. Die notwendige Menge an Basen kann auch dadurch in das Reaktionssystem eingebracht werden, daß man beispielsweise Maleinsäure oder Itaconsäure, die partiell (20 bis 60 mol-%) mit einer Alkalimetallbase neutralisiert sind, verwendet. Vorzugsweise legt man die Verbindungen der Formel II, Kalziumhydroxyd und Maleinsäure oder ltaconsäure in einem Reaktionsgefäß vor und gibt unter guter Durchmischung Natronlauge, Kalilauge oder ein tertiäres Amin während der Reaktion zu, so daß der pH-Wert in dem Bereich von 9 bis 13 gehalten wird.

Die Umsetzung wird in dem Temperaturbereich von 50 bis 150, vorzugsweise von 60 bis 100°C durchgeführt. Sofern man oberhalb des Siedepunkts der Reaktionsmischung arbeitet, führt man die Reaktion in druckdicht verschlossenen Vorrichtungen durch, z.B. in Autoklaven, die mit einem Rührer versehen sind. Nach Beendigung der Umsetzung ist es in den meisten Fällen erforderlich, die eingesetzte Erdalkalimetallverbindung von den Alkylmono- oder Alkylpolyglucosidethercarboxylaten zu trennen. Zu diesem Zweck leitet man vorzugsweise Kohlendioxid in das Reaktionsgemisch ein und fällt dadurch die Erdalkalimetallionen als Carbonate aus der wäßrigen Lösung. Die Verbindungen der Formel I können anschließend aus der wäßrigen Lösung durch Eindampfen oder Ausfällen gewonnen werden. Sofern relativ geringe Mengen an Nebenprodukten nicht stören, können die wäßrigen Reaktionslösungen, die von den Erdalkalimetallionen weitgehend befreit sind, direkt verwendet werden. Man kann jedoch auch die Alkylmono- und Alkylpolyglucosidethersuccinate aus den wäßrigen Lösungen durch Zusatz von beispielsweise Methanol fraktioniert ausfällen und auf diese Weise reinigen. Ebenso ist eine Reinigung der Verbindungen der Formel I durch fraktionierte Kristallisation aus wäßriger Lösung möglich.

Die Alkyl(poly)glucosidethercarboxylate der Formel I werden als Zusatz zu phosphatfreien oder phosphatarmen Wasch- und Reinigungsmitteln in Mengen von 0,1-20 Gew.-%, bezogen auf die jeweilige Formulierungen, verwendet. Unter Wasch- und Reinigungsmitteln mit einem reduzierten Phosphatgehalt soll verstanden werden, daß diese Formulierungen einen gesamten Phosphatgehalt von weniger als 25 Gew.-% Natriumtriphosphat aufweisen. Die Verbindungen der Formel I können der jeweiligen Wasch- oder Reinigungsmittelformulierung in Form eines Granulats, einer Paste, einer hochviskosen Masse, als Dispersion oder auch als Lösung in wasser zugesetzt werden. Sie können jedoch auch an der Oberfläche von Stellmitteln, z.B. Natriumsulfat oder Gerüststoffen (Zeolithen oder Bentoniten) sowie anderen festen Hilfsstoffen der Waschmittelformulierungen adsorbiert werden.

Die Zusammensetzung von Waschmittelformulierungen kann sehr unterschiedlich sein. Gleiches gilt für die Zusammensetzung von Reinigungsmittelformulierungen. Wasch- und Reinigungsmittelformulierungen enthalten üblicherweise Tenside und gegebenenfalls Builder. Diese Angaben gelten sowohl für flüssige als auch für pulverförmige Wasch- und Reinigungsmittelformulierungen. Beispiele für die Zusammensetzung von Waschmittelformulierungen, die in Europa, in den USA und in Japan gebräuchlich sind, findet man beispielsweise in Chemical and Engn. News, Band 67, 35 (1989) tabellarisch dargestellt sowie in Ullmanns Encyklopädie der technischen Chemie, Verlag Chemie, Weinheim 1983, 4. Auflage, Seiten 63-160.

Waschmittelformulierungen sind pulverförmig oder flüssig. Die pulverförmigen Waschmittel können regional und gemäß dem speziellen Anwendungszweck verschieden zusammengesetzt sein.

Universalhaushaltswaschmittel für Trommelwaschmaschinen, wie sie in Europa weit verbreitet sind, enthalten gewöhnlich 5 bis 10 Gew.% Aniontenside; 1 bis 5 Gew.% nichtionische Tenside; 1 bis 5 Gew.% Schaumregulatoren, wie Silikonöle oder Seifen; 0 bis 40 Gew.% Enthärtungsmittel, wie Soda oder Pentanatriumtriphosphat, das durch die erfindungsgemäßen Verbindungen teilweise oder ganz ersetzt werden kann; 0 bis 30 Gew.% Ionenaustauscher, wie Zeolith A; 2 bis 7 Gew.% Natriumsilikate als Korrosionsinhibitoren; 10 bis 30 Gew.% Bleichmittel, wie Natriumperborat oder Natriumpercarbonat; 0 bis 5 Gew.% Bleichaktivatoren, wie Tetraacetylethylendiamin, Pentaacetylglucose, Hexaacetylsorbit oder Acyloxibenzolsulfonat; Stabilisatoren, wie Magnesiumsilikat oder Ethylendiamintetraacetat; Vergrauungsinhibitoren, wie Carboximethylcellulose, Methyl- und Hydroxialkylcellulosen, mit Vinylacetat gepfropfte Polyglykole, oligomere und polymere Terephthalsäure/Ethylenglykol/Polyethylenglykol-Ester; Enzyme; optische Aufheller; Duftstoffe; Weichmacher; Farbstoffe und Stellmittel.

Im Gegensatz hierzu sind die Heavy Duty Detergents, die in den USA, Japan und diesen Ländern benachbarten Staaten in den Bottichwaschmaschinen verwendet werden, meist frei von Bleichmitteln, ihr Anteil an Aniontensiden ist dafür zwei bis dreimal so hoch, sie enthalten mehr Waschalkalien, wie Soda und Natriumsilikate (in der Regel bis zu 25 Gew.%) und natürlich fehlen ihnen auch die Bleichaktivatoren und Bleichstabilisatoren. Die Gehaltsangaben für Tenside und andere Inhaltsstoffe können sich noch beträchtlich erhöhen, wenn es sich um sogenannte Waschmittelkonzentrate handelt, die stellmittelfrei oder stellmittelarm in den Handel kommen. Fein- und Buntwaschmittel, Wollwaschmittel und Mittel für die manuelle Wäsche enthalten ebenfalls meist kein Bleichmittel und geringe alkalische Bestandteile bei entsprechend erhöhtem Tensidanteil.

Waschmittel für den gewerblichen Sektor sind auf die speziellen Verhältnisse des industriellen Waschens zugeschnitten (weiches Wasser, kontinuierliches Waschen), die es gestatten, schwerpunktmäßig auf die Art des Waschguts und der Verschmutzung einzugehen. Es werden daher Kombinationen verwendet, in denen ein Bestandteil vorherrscht oder andere ganz fehlen, die bei Bedarf getrennt zudosiert werden. Deshalb variieren die Bestandteile Tenside, Builder (Gerüststoffe), Alkalien und Bleichmittel dieser Waschmittel in weiten Grenzen.

Geeignete anionische Tenside für die vorgenannten Pulverwaschmittel sind beispielsweise Natriumalkylbenzolsulfonate, Fettalkoholsulfate und Fettalkoholpolyglykolethersulfate. Einzelne Verbindungen dieser Art sind beispielsweise C₈- bis C₁₂-Alkylbenzolsulfonate, C₁₂- bis C₁₆-Alkansulfonate, C₁₂- bis C₁₆-Alkylsulfate, C₁₂- bis C₁₆-Alkylsulfosuccinate und sulfatierte ethoxylierte C₁₂- bis C₁₆-Alkanole. Als anionische Tenside eignen sich außerdem sulfatierte Fettsäurealkanolamine, Fettsäuremonoglyceride oder Umsetzungsprodukte von 1 bis 4 Mol Ethylenoxid mit primären oder sekundären Fettalkolen oder Alkylphenolen. Weitere geeignete anionische Tenside sind Fettsäureester bzw. Fettsäureamide von Hydroxy- oder Aminocarbonsäuren bzw. -sulfonsäuren, wie beispielsweise die Fettsäuresarkoside, -glykolate, -lactate, -tauride oder -isothionate. Die anionischen Tenside können in Form der Natrium-, Kalium- und Ammoniumsalze sowie als lösliche Salze organischer Basen, wie Mono-, Di- oder Triethanolamin oder andere substituierter Amine vorliegen. Zu den anionischen Tensiden gehören auch die üblichen Seifen, d.h. die Alkalisalze der natürlichen Fettsäuren.

Als nichtionische Tenside (Nonionics) sind z.B. Anlagerungsprodukte von 3 bis 40, vorzugsweise 4 bis 20 Mol Ethylenoxid an 1 Mol Fettalkohol, Alkylphenol, Fettsäure, Fettamin, Fettsäureamid oder Alkansulfonamid verwendbar. Besonders wichtig sind die Anlagerungsprodukte von 5 bis 16 Mol Ethylenoxid an Kokos- oder Talgfettalkohole, an Oleylalkohol oder an synthetische Alkohole mit 8 bis 18, vorzugsweise 12 bis 18 C-Atomen, sowie an Mono- oder Dialkylphenole mit 6 bis 14 C-Atomen in den Alkylresten. Neben diesen wasserlöslichen Nonionics sind aber auch nicht bzw. nicht vollständig wasserlösliche Polyglykolether mit 1 bis 4 Ethylenglykoletherresten im Molekül von Interesse, insbesondere wenn sie zusammen mit wasserlöslichen nichtionischen oder anionischen Tensiden eingesetzt werden.

Weiterhin sind als nichtionische Tenside die wasserlöslichen, 20 bis 250 Ethylenglykolethergruppen und 10 bis 100 Propylenglykolethergruppen enthaltenden Anlagerungsprodukte von Ethylenoxid an Polypropylenglykolether, Alkylendiaminopolypropylenglykol und Alkylpolypropylenglykole mit 1 bis 10 C-Atomen in der Alkylkette brauchbar, in denen die Polypropylenglykoletherkette als hydrophober Rest fungiert.

Auch nichtionische Tenside vom Typ der Aminoxide oder Sulfoxide sind verwendbar.

Das Schaumvermögen der Tenside läßt sich durch Kombination geeigneter Tensidtypen steigern oder verringern. Eine Verringerung läßt sich ebenfalls durch Zusätze von nichttensidartigen organischen Substanzen erreichen.

Ein weiterer wichtiger Bestandteil in Waschmittelformulierungen sind Inkrustierungsinhibitoren. Bei diesen Stoffen handelt es sich beispielsweise um Homopolymerisate der Acrylsäure, Methacrylsäure und Maleinsäure oder um Copolymerisate, z.B. Copolymerisate aus Maleinsäure und Acrylsäure, Copolymerisate aus Maleinsäure und Methacrylsäure bzw. aus Copolymerisaten von a) Acrylsäure und/oder Methacrylsäure mit b) Acrylsäureestern, Methacrylsäureestern, Vinylestern, Allylestern, Itaconsäureestern, Itaconsäure, Methylenmalonsäure, Methylenmalonsäureester, Crotonsäure und Cotonsäureester. Außerdem kommen Copolymere aus Olefinen und C₁- bis C₄-Alkylvinylethern in Betracht. Das Molekulargewicht der Homo- und Copolymerisate beträgt 1 000 bis 100 000. Die Inkrustationsinhibitoren werden in einer Menge von 0,5 bis 10 Gew.% in Waschmitteln verwendet, wobei sie in nicht neutralisierter Form, als Alkali- oder Ammoniumsalz sowie in partiell neutralisierter Form, z.B. Neutralisation von 40 bis 60 % der Carboxylgruppen, eingesetzt werden.

Weitere Mischungsbestandteile von Waschmitteln können auch monomere, oligomere und polymere Phosphonate, Ethersulfonate auf der Basis von ungesättigten Fettalkoholen, z.B. Oleylalkoholethoxylatbutylether und deren Alkalisalze sein. Diese Stoffe können z.B. mit Hilfe der Formel RO(CH₂CH₂O)n-C₄H₈-SO₃Na, in der n = 5 bis 40 und R = Oleyl ist, charakterisiert werden.

Die oben beschriebenen Alkyl(poly)glucosidethercarboxylate der Formel I können auch als Zusatz zu Flüssigwaschmitteln verwendet werden. Die Flüssigwaschmittel enthalten als Abmischkomponente flüssige oder auch feste Tenside, die in der Waschmittelformulierung löslich oder zumindest dispergierbar sind. Als Tenside kommen hierfür die Produkte in Betracht, die auch in pulverförmigen Waschmitteln eingesetzt werden sowie flüssige Polyalkylenoxide bzw. polyalkoxylierte Verbindungen. Falls die Copolymerisate mit den übrigen Bestandteilen des Flüssigwaschmittels nicht direkt mischbar sind, kann man mit Hilfe geringer Menge an Lösungsvermittlern, z.B. Wasser oder eines mit Wasser mischbaren organischen Lösemittel, z.B. Isopropanol, Methanol, Ethanol, Glykol, Diethylenglykol oder Triethylenglykol oder entsprechende Propylenglykole, homogene Mischungen herstellen. Die Tensidmenge in Flüssigwaschmitteln beträgt 4 bis 50 Gew.%, bezogen auf die gesamte Formulierung, da auch bei den Flüssigwaschmitteln je nach den regionalen Marktgegebenheiten oder dem Anwendungszweck die Anteile der Bestandteile in weiten Grenzen variieren.

Die Flüssigwaschmittel können Wasser in Mengen von 10 bis 60, vorzugsweise 20 bis 50 Gew.% enthalten. Sie können aber auch wasserfrei sein.

Wasserfreie Flüssigwaschmittel können auch Peroxoverbindungen zum Bleichen in suspendierter oder dispergierter Form enthalten. Als Peroxoverbindungen seien z.B. genannt: Natriumperborat, Peroxocarbonsäuren und Polymere mit teilweise peroxohaltigen Gruppen. Außerdem können die Flüssigwaschmittel gegebenenfalls Hydrotrope enthalten. Hierunter werden Verbindungen verstanden wie 1,2-Propandiol, Cumolsulfonat und Toluolsulfonat. Falls derartige Verbindungen zur Modifizierung der Flüssigwaschmittel eingesetzt werden, beträgt ihre Menge, bezogen auf das Gesamtgewicht des Flüssigwaschmittels, 2 bis 5 Gew.%. In vielen Fällen hat sich zur Modifizierung von pulverförmigen und flüssigen Waschmitteln auch ein Zusatz von Komplexbildnern als vorteilhaft erwiesen. Komplexbildner sind beispielsweise Ethylendiamintetraessigsäure, Nitrilotriacetat und Isoserindiessigsäure sowie Phosphonate, wie Aminotrismethylenphosphonsäure, Hydroxyethandiphosphonsäure, Ethylendiamintetraethylenphosphonsäure und deren Salze. Die Komplexbildner werden in Mengen von 0 bis 10 Gew.%, bezogen auf die Waschmittel, eingesetzt. Die Waschmittel können außerdem Zitrate, Di- oder Triethanolamin, Trübungsmittel, optische Aufheller, Enzyme, Parfümöle und Farbstoffe enthalten. Diese Stoffe sind, falls sie zur Modifizierung der Flüssigwaschmittel verwendet werden, zusammen in Mengen bis zu 5 Gew.% anwesend. Die Waschmittel sind vorzugsweise phosphatfrei. Sie können jedoch auch Phosphate enthalten, z.B. Pentanatriumtriphosphat und/oder Tetrakaliumpyrophosphat. Falls Phosphate eingesetzt werden, beträgt der Anteil der Phosphate an der Gesamtformulierung des Waschmittels bis 25 Gew.%.

Die Alkyl(poly)glucosidethercarboxylate eignen sich außerdem als Zusatz beim Nachbehandeln von synthetische Fasern enthaltendem Textilgut, z.B. zur Erzeugung von soil release-Effekten. Sie werden zu diesem Zweck dem letzten Spülbad eines Waschmaschinenzyklus zugesetzt, wobei der Zusatz entweder zusammen mit einem an dieser Stelle üblicherweise angewendeten Wäscheweichspüler erfolgen kann oder - falls ein Weichspüler nicht erwünscht ist - allein anstelle des Weichspülers. Die Einsatzmengen betragen 0,01 bis 0,3 g/l Waschflotte. Die Verwendung der Verbindungen der Formel I im letzten Spülbad eines Waschmaschinenzyklus hat den Vorteil, daß die Wäsche beim nächsten Waschzyklus weit weniger von abgelösten Schmutzteilchen, die in der Waschflotte vorhanden sind, angeschmutzt wird als ohne den Zusatz des Vergrauungsinhibitors bei der vorausgegangen Wäsche.

Die erfindungsgemäßen Verbindungen der Formel I können auch mit anderen bekannten Waschmitteladditiven (wie z.B. Inkrustationsinhibitoren, Vergrauungsinhibitoren, Clay-Dispergatoren und Stoffen, die die Primärwaschwirkung verstärken, Farbübertragungsinhibitoren, Bleichaktivatoren) in Pulver- und Flüssigwaschmitteln (phosphathaltig und phosphatfrei) synergistische Effekte hervorrufen, bei denen nicht nur die Vergrauungsinhibierung, sondern auch die Wirkung des anderen Waschmitteladditivs verstärkt werden kann. Die Alkyl(poly)glucosidethercarboxylate sind vor allem geeignet zur Entfernung von Partikelschmutz, z.B. von Clay. Sie sind biologisch abbaubar. Bemerkenswerterweise besitzen C₈- bis C₁₈-Alkyl(poly)glucosidethercarboxylate der Formel I mit y = 0 und die entsprechenden alkoxylierten Verbindungen mit y = 2 bis 10 und R¹ = H gleichzeitig Tensid- und Buildereigenschaften.

Die in den Beispielen angegebenen Teile sind Gewichsteile, die Angaben in Prozent beziehen sich auf das Gewicht der Stoffe. Der Grad der Glucosidierung x in Formel I wurde durch Flüssigkeitschromatographie (HPLC) bestimmt.

### Beispiele

### Beispiel 1

In einem mit einem Rührer und einem Rückflußkühler ausgestatteten Reaktionsgefäß wurden 90 g eines Hydroxyethylpolyglucosids mit einem Glucosidierungsgrad x von 1,3 in 700 ml Wasser gelöst. Hydroxyethylpolyglucosid wurde nach der in Tenside Detergents Band 10, Heft 1, Seite 2 (1973) gegebenen Vorschrift hergestellt. Man fügt dann 119 g (1,6 mol) Kalziumhydroxyd zu und erhitzt das Reaktionsgemisch auf 60°C. Sobald diese Temperatur erreicht ist, gibt man unter kräftigem Rühren 223 g (1,9 mol) Maleinsäure innerhalb von 1 h portionsweise zu und hält außerdem durch Zugabe von insgesamt 80 g 50%iger wäßriger Natronlauge den pH-Wert des Reaktionsgemisches in dem Bereich 11 bis 11,5. Nach Zugabe der Maleinsäure und der Natronlauge wird das Reaktionsgemisch noch 6 h auf 80°C erwärmt. Danach leitet man bei einer Temperatur von 60 bis 70°C insgesamt 75 g Kohlendioxid ein, wobei der pH-Wert durch Zugabe von 50%iger wäßriger Natronlauge bei 10 gehalten wird. Danach wird das Kalziumcarbonat abfiltriert und mit 200 ml Wasser gewaschen. Man erhält eine 35%ige wäßrige Lösung des Natriumsalzes von Hydroxyethylpolyglucosidethersuccinat der Formel I mit
- R =: 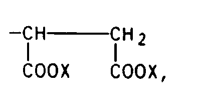
- R¹=: H,
- y=: 1,
- x=: 1,3 und
- z≈: 3,1.

Die 35%ige wäßrige Lösung enthielt als Nebenkomponenten die Natriumsalze von Maleinsäure (1,3%), Fumarsäure (3,1%) und von Natriumcarbonat (1,9%).

### Beispiel 2

Beispiel 1 wurde mit den Ausnahmen wiederholt, daß man Kalziumhydroxyid in einer Menge von 96,2 g (1,3 mol) und Maleinsäure in einer Menge von 174 g (1,5 mol) einsetzte. Man erhielt eine 35%ige wäßrige Lösung, die als Nebenprodukte Natriummaleinat (1,0%), Natriumfumarat (2,2%) und Natriumcarbonat (1,9%) enthielt. In Formel I bedeutet dann z ≈ 2,3.

### Beispiel 3

Beispiel 1 wurde mit den Ausnahmen wiederholt, daß man Maleinsäure in einer Menge von 139 g (1,2 mol) und Kalziumhydroxyd in einer Menge von 81 g (1,1 mol) einsetzte. Man erhielt nach der Aufarbeitung des Reaktionsgemisches eine 32%ige wäßrige Lösung mit 0,9% Natriummaleinat, 1,5% Natriumfumarat und 2% Natriumcarbonat als Nebenkomponenten. In Formel I steht z dann für ≈ 1,7.

### Beispiel 4

Beispiel 1 wurde mit den Ausnahmen wiederholt, daß man 160 g einer 70%igen wäßrigen Lösung eines Alkylpolyglucosids auf Basis von C₁₀-C₁₂-Alkoholen und einem Glucosidierungsgrad x = 2,2 mit 174 g (1,5 mol) Maleinsäure und 111 g (1,5 mol) Kalziumhydroxyd umsetzte. Nach der Aufarbeitung erhielt man eine 40%ige wäßrige Lösung eines Alkylpolyglucosidethersuccinatnatriumsalzes der Formel I mit
- R=: C₁₀/C₁₂-Alkyl
- y=: 0
- z≈: 2,8
- x=: 2,2 und
- R²=: 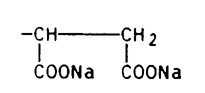
Die wäßrige Lösung enthielt als Nebenkomponenten 2,8% Natriummaleinat, 3,2% Natriumfumarat sowie 1,1% Natriumcarbonat.

### Anwendungstechnische Beispiele

Die erfindungsgemäßen Verbindungen eignen sich insbesonders in Waschmitteln zur Entfernung von Partikelschmutz von Gewebeoberflächen. Diese Eigenschaft von Polyelektrolyten kann beispielsweise mit Hilfe der sogenannten Clay-Dispergierung quantitativ erfaßt werden.

Die Stabilisierung der nach der Ablösung der Partikel von der Gewebeoberfläche entstehenden Dispersion ist eine wichtige Aufgabe dieser Polyelektrolyte. Der stabilisierende Einfluß der anionischen Dispergiermittel ergibt sich dadurch, daß infolge von Adsorption von Dispergiermittelmolekülen auf der Feststoffoberfläche deren Oberflächenladung vergrößert und die Abstoßungsenergie erhöht wird. Weitere Einflußgrößen auf die Stabilität einer Dispersion sind ferner u.a. sterische Effekte, Temperatur, pH-Wert und die Elektrolytkonzentration.

Mit dem im folgenden beschriebenen Clay-Dispergiertest (CD-Test) kann auf einfache Weise die Dispergierfähigkeit verschiedener Polyelektrolyte beurteilt werden.

### CD-Test

Als Modell für partikulären Schmutz wird feingemahlener China-Clay SPS 151 benutzt. 1 g Clay wird unter Zusatz von 1 ml einer 0,1 %igen Natrium-salzlösung des Polyelektrolyten in 98 ml Wasser 10 min in einem Standzylinder (100 ml) intensiv dispergiert. Sofort nach dem Rühren nimmt man aus der Mitte des Standzylinders eine Probe von 2,5 ml und bestimmt nach dem Verdünnen mit Wasser auf 25 ml die Trübung der Dispersion mit einem Turbidimeter. Nach 30- bzw. 60minütiger Standzeit der Dispersion werden erneut Proben genommen und wie oben die Trübung bestimmt. Die Trübung der Dispersion wird in NTU (nephelometric turbidity units) angegeben. Je weniger sich die Dispersion während der Lagerung absetzt, um so höher sind die gemessenen Trübungswerte und um so stabiler ist die Dispersion.

Als zweite physikalische Meßgröße wird die Dispersionskonstante τ bestimmt, die das zeitliche Verhalten des Sedimentationsprozesses beschreibt. Da der Sedimentationsprozess annähernd durch ein monoexponentielles Zeitgesetz beschrieben werden kann, gibt τ die Zeit an, in der die Trübung auf 1/e-tel des Ausgangszustandes zum Zeitpunkt t = 0 abfällt.

Als Polyelektrolyte wurden die gemäß den Beispielen 1 bis 4 hergestellten Alkylpolyglucosidether-Natrium-Succinate getestet. Die dabei erhaltenen Ergebnisse sind in der folgenden Tabelle angegeben.

| Beispiel Nr. | Alkylpolyglucosidethersuccinate gemäß Beispiel | Clay-Dispergiertest Trübung nach Lagerung | | | Disp.konst. τ |
|---|---|---|---|---|---|
| | | sofort | 30 min | 60 min | |
| 5 | 1 | 790 | 600 | 560 | 253,7 |
| 6 | 2 | 700 | 620 | 530 | 220,3 |
| 7 | 3 | 780 | 650 | 500 | 141,2 |
| 8 | 4 | 670 | 400 | 300 | 76,2 |
| Vergleichs-beispiel | ohne Zusatz | 600 | 37 | 33 | 41,4 |

Die Trübungswerte sind angegeben in NTU (nephelometric turbidity units).

Die Alkylpolyglucosidethersuccinate gemäß den Beispielen 1 bis 4 besitzen im Vergleich zum Vergleichsversuch ein deutlich verbessertes Dispergiervermögen für Clay. Es sind sowohl die gemessenen Trübungswerte höher (bessere Dispergierung) als auch die Dispergierkonstanten (höhere Stabilität der Dispersion).

## Patentansprüche

1. Alkylmono- und Alkylpolyglucosidethercarboxylate der Formel 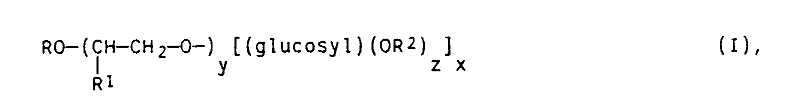 in der
R = C₁- bis C₂₄-Alkyl, C₁- bis C₂₄-Alkylphenyl, Hydroxy-C₂- bis C₆-alkyl, Hydroxy-C₁- bis C₂₄-alkylphenyl, 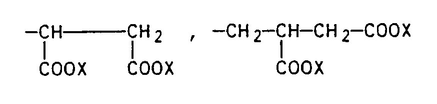 oder H,
R¹= H oder Methyl,
R²= 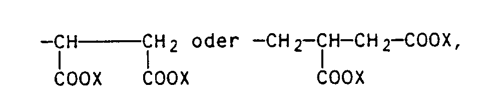
X= Wasserstoff-, Alkalimetall-, Ammonium- und/oder substituiertes Ammoniumäquivalent,
x= 1 bis 10,
y= 1 bis 10 und
z= 1 bis 4
bedeuten.

2. Verfahren zur Herstellung von Alkylmono- und Alkylpolyglucosidethercarboxylaten der Formel I nach Anspruch 1, dadurch gekennzeichnet, daß man Alkylmono- oder Alkylpolyglucoside der Formel 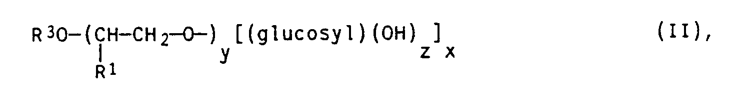 in der
R³= C₁- bis C₂₄-Alkyl, C₁- bis C₂₄-Alkylphenyl, Hydroxy-C₂- bis C₆-alkyl, Hydroxy-C₂- bis C₂₄-alkylphenyl oder Wasserstoff,
R¹= H oder Methyl,
x= 1 bis 10,
y= 1 bis 10 und
z= 1 bis 4
bedeuten, durch Reaktion mit Maleinsäure und/oder Itaconsäure in wäßrigem Medium in Gegenwart von mindestens 30 mol-% Erdalkalimetallionen, bezogen auf die genannten Dicarbonsäuren, bei pH-Werten von 9 bis 13 und Temperaturen von 50 bis 150°C zu Verbindungen der Formel I verethert.

3. Verwendung der Alkylmono- und Alkylpolyglucosidethercarboxylate nach Anspruch 1 als Zusatz zu phosphatfreien oder phosphatarmen Wasch- und Reinigungsmitteln in Mengen von 0,1 bis 20 Gew.-%, bezogen auf die jeweiligen Formulierungen.

## Claims

1. An alkyl mono- or polyglucoside ether carboxylate of the formula 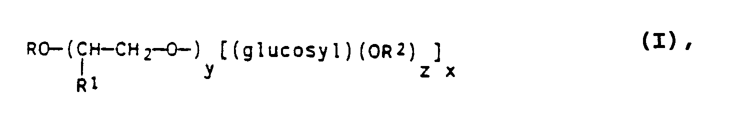 where
R is C₁- to C₂₄-alkyl, C₁- to C₂₄-alkylphenyl, hydroxy-C₂-to C₆-alkyl, hydroxy-C₁- to C₂₄-alkylphenyl, 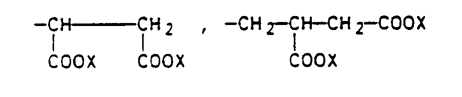 or H,
R¹ is H or methyl, 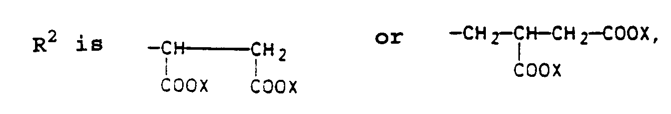
X is a hydrogen, alkali metal, ammonium and/or substituted ammonium equivalent,
x is from 1 to 10,
y is from 0 to 10, and
z is from 1 to 4.

2. A process for the preparation of an alkyl mono-or polyglucoside ether carboxylate of the formula I as claimed in claim 1, which comprises etherifying an alkyl mono- or polyglucoside of the formula 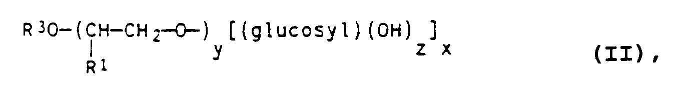 where
R³ is C₁- to C₂₄-alkyl, C₁- to C₂₄-alkylphenyl, hydroxy-C₂-to C₆-alkyl, hydroxy-C₂- to C₂₄-alkylphenyl or hydrogen,
R¹ is H or methyl,
x is from 1 to 10,
y is from 1 to 10, and
z is from 1 to 4,
by reaction with maleic acid and/or itaconic acid in aqueous medium in the presence of 30 mol-% or more of alkaline earth metal ions, based on said dicarboxylic acid, at a pH of from 9 to 13 and at from 50 to 150°C.

3. The use of an alkyl mono- or polyglucoside ether carboxylate as claimed in claim 1 as an additive to phosphate-free or low-phosphate detergents and cleaners in an amount of from 0.1 to 20% by weight, based on the particular formulation.

## Revendications

1. Ether-carboxylates d'alkylmono- et alkylpolyglucosides de formule 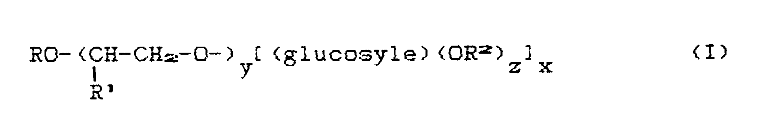 dans laquelle les symboles ont les significations suivantes:
R = Groupement alkyle en C₁-C₂₄, (alkyle en C₁-C₂₄)phényle, hydroxyalkyle en C₂-C₆, (hydroxyalkyle en C₁-C₂₄)phényle, 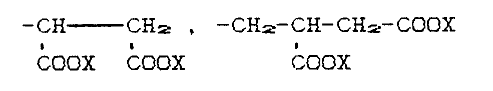 ou atome d'hydrogène,
R¹= Atome d'hydrogène ou groupement méthyle,
R²= 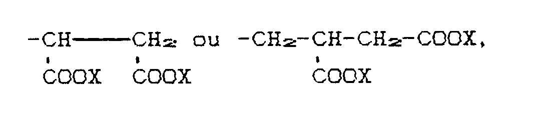
X = Atome d'hydrogène, de métal alcalin, ion ammonium et/ou ammonium substitué,
x = 1 à 10,
y = 1 à 10 et
z = 1 a 4.

2. Procédé de préparation d'éther-carboxylates d'alkylmono- et alkylpolyglucosides de formule I selon la revendication 1, caractérisé en ce que l'on éthérifie en composés de formule I des alkylmono- ou alkylpolyglucosides de formule 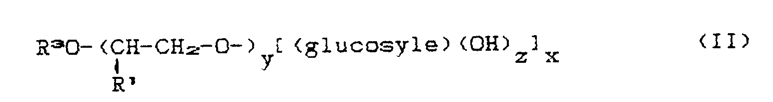 dans laquelle
R³ représente un groupement alkyle en C₁-C₂₄, (alkyle en C₁-C₂₄)phényle, hydroxyalkyle en C₂-C₆, (hydroxyalkyle en C₂-C₂₄)phényle ou un atome d'hydrogène
R¹ représente un atome d'hydrogène ou un reste méthyle,
x= 1 à 10,
y= 1 à 10 et
z= 1 à 4,
par réaction avec de l'acide maléique et/ou de l'acide itaconique en milieu aqueux, en présence d'au moins 30% en moles d'ions de métal alcalino-terreux par rapport auxdits acides dicarboxyliques, à des pH de 9 à 13 et à des températures de 50 à 150°C.

3. Utilisation des éther-carboxylates d'alkylmono- et alkylpolyglucosides selon la revendication 1 comme additifs à des produits de lavage et de nettoyage dépourvus de phosphates ou pauvres en phosphates, dans des proportions de 0,1 à 20% en poids par rapport aux compositions en question.
